# EUROPÄISCHE PATENTANMELDUNG

(11) **EP 2 266 657 A1**
(43) Veröffentlichungstag der Anmeldung: **29.12.2010**
(21) Anmeldenummer: 10157294.9
(22) Anmeldetag: 23.03.2010
(51) Int. Cl.: A61N 1/05

(54) **Elektrodensonde zur medizinischen Anwendung**

(30) Priorität: 23.06.2009 US 219434 P
(71) Anmelder: Biotronik CRM Patent AG, 6341 Baar (CH)
(72) Erfinder: Trip, Erik, 7975, Uffelte (NL); Flach, Erhard, 12305, Berlin (DE); Schnittker, Christian, 10407, Berlin (DE); Schurr, Marc, 10179, Berlin (DE)
(74) Vertreter: Lindner-Vogt, Karin L.

(57) **Zusammenfassung**

Die Erfindung betrifft; eine Elektrodensonde zur medizinischen Anwendung, welche einen schlauchartigen, flexiblen Sondenkörper, eine am distalen Ende des Sondenkörpers angeordnete Elektrode, sowie eine im Sondenkörper geführte elektrische Zuleitung zur Elektrode umfasst, und sich dadurch auszeichnet, dass zumindest ein Teil eines distalen Endabschnitts des Sondenkörpers durch einen mit diesem gekoppelten Bewegungsmechanismus von einem ersten Zustand in einen in Bezug auf den ersten Zustand radial verbreiterten zweiten Zustand überführbar ist. Insbesondere ist wenigstens ein Teil des distalen Endabschnitts bei einer Relativbewegung zwischen der elektrischen Zuleitung und dem Sondenkörper durch eine von der Elektrode auf den distalen Endabschnitt übertragene Kraft zu einer radial verbreiterten Wulst verformbar. Im Falle einer Einschraubelektrode wird der distale Endabschnitts beim Einschrauben der Einschraubelektrode in das Körpergewebe durch eine vom Körpergewebe auf den distalen Endabschnitt ausgeübte Kraft und/oder durch eine von der Einschraubelektrode über ein Gegenlager auf den distalen Endabschnitt ausgeübte Kraft zu einer radial verbreiterten Wulst verformt.

## Beschreibung

Die Erfindung liegt auf dem Gebiet der medizinischen Sonden und betrifft nach ihrer Gattung eine implantierbare Elektrodensonde mit einem schlauchartigen, flexiblen Sondenkörper, einer am distalen Ende des Sondenkörpers angeordneten Elektrode, sowie einer im Sondenkörper geführten elektrischen Zuleitung zur Elektrode. Gattungsgemäße Elektrodensonden werden beispielsweise als Herzschrittmacherelektroden routinemäßig eingesetzt. In der Patentliteratur sind sie vielfach beschrieben. Lediglich beispielhaft sei in diesem Zusammenhang auf die Druckschriften DE 10 2005 039 040 A1, DE 198 00 697 A1, DE 20 2006 020 517 U1 und EP 88730200.8 verwiesen.

Im Körper implantierbare Sonden, insbesondere Elektrodensonden, sollen einen möglichst geringen Sondendurchmesser haben, um einerseits eine komfortable Passage durch die Einführschleuse und Blutgefäße bis zum gewünschten Implantationsort sicherzustellen und andererseits die Verwendung kleiner Einführbestecke zu ermöglichen. Jedoch erhöhen Sonden mit geringem Durchmesser die Gefahr einer Perforation des Körpergewebes am Implantationsort.

Wie dem Fachmann weiterhin bekannt ist, können Wirkstoffe am Implantationsort durch die Sonde, insbesondere Elektrodensonde, abgegeben werden. Beispielsweise ist in den Druckschriften US 5,571,163 A und US 5,324,324 A1 vorgeschlagen, die Elektrodenspitze mit entzündungshemmenden Medikamenten zu beschichten, um den durch die Sonde ausgelösten Gewebereizungen lokal entgegen zu wirken. Nachteilig bei Elektrodensonden mit einem relativ geringen Sondendurchmesser ist die geringe Sondenoberfläche, die für die Wirkstoffabgabe zur Verfügung steht. Zudem erstreckt sich das Wirkstoffreservoir bei verringertem Sondendurchmesser und gleich bleibendem Wirkstoffgehalt über einen größeren Abschnitt der Sonde, so dass der Wirkstoff zum Teil erst in relativ weiter Entfernung zum Implantationsort freigesetzt wird, was dem therapeutischen Nutzen des abgegebenen Wirkstoffs nachträglich sein kann.

In der praktischen Anwendung von Elektrodensonden, insbesondere solchen mit der Möglichkeit einer Wirkstoffabgabe nahe des Implantationsorts, ist zur Lösung dieses Zielkonflikts ein Kompromiss hinsichtlich des Durchmessers der Elektrodensonde zu finden, der optimal sowohl hinsichtlich einer einfachen Implantierbarkeit der Sonde als auch für eine lokale Wirkstoffabgabe durch die Sonde ist.

Demgegenüber besteht die Aufgabe der vorliegenden Erfindung darin, eine implantierbare Elektrodensonde zur Verfügung zu stellen, die einerseits einen relativ geringen Sondendurchmesser aufweist, um ein möglichst komfortables Implantieren der Elektrodensonde sicherzustellen, andererseits aber auch eine hohe Perforationssicherheit bietet und eine effiziente Wirkstoffabgabe am Implantationsort, d. h. an der Stelle der durch die Sonde ausgelösten Gewebereizung, ermöglicht.

Diese und weitere Aufgaben werden nach dem Vorschlag der Erfindung durch eine implantierbare Elektrodensonde mit den Merkmalen des unabhängigen Anspruchs gelöst. Vorteilhafte Ausgestaltungen der Erfindung sind durch die Merkmale der Unteransprüche angegeben.

Erfindungsgemäß ist eine Elektrodensonde zur medizinischen Anwendung, insbesondere eine Herzschrittmacherelektrode, eine Elektrode zur Nervenstimulation, eine Elektrode zur Messung von Hirnpotentialen oder dergleichen, gezeigt. Die Elektrodensonde umfasst gattungsgemäß einen schlauchartigen, flexiblen Sondenkörper, eine am distalen Ende des Sondenkörpers angeordnete Elektrode, beispielsweise Spitzenelektrode, sowie eine im Sondenkörper geführte elektrische Zuleitung zur Elektrode. Weiterhin können entlang des Sondenköpers ein oder mehrere weitere elektrische Kontakte angebracht sein. Gemäß vorliegender Erfindung zeichnet sich die Elektrodensonde in wesentlicher Weise dadurch aus, dass zumindest ein Teil eines distalen Endabschnitts des Sondenkörpers, an dessen Ende die Elektrode angeordnet ist und welcher somit zur Elektrode unmittelbar benachbart ist, durch einen mit dem distalen Endabschnitt in Wirkverbindung stehenden Bewegungsmechanismus von einem ersten Zustand in einen in Bezug auf den ersten Zustand radial verbreiterten zweiten Zustand überführbar ist. Im zweiten Zustand ist der distale Endabschnitt des Sondenkörpers in Bezug auf eine radiale Abmessung des übrigen Sondenkörpers und der am distalen Ende des Sondenkörpers angeordneten Elektrode radial verbreitert.

Die Elektrodensonde kann somit in vorteilhafter Weise mit einem dünnen distalen Endabschnitt implantiert werden, wobei durch die geringere radiale Abmessung des distalen Endabschnitts des Sondenkörpers eine komfortable Passage durch die Einführschleuse und Gefäße gewährleistet ist. Andererseits kann der distale Endabschnitt des Sondenkörpers am Implantationsort radial verbreitert werden, wobei einerseits ein ausreichender Perforationsschutz zum Verhindern einer Gewebeperforation durch die Elektrode und andererseits eine relativ große Fläche für die lokale Abgabe eines Wirkstoffs am Implantationsort zur Verfügung gestellt werden. Zudem kann der Wirkstoff durch die räumliche Nähe bzw. Kontakt des verbreiterten Bereichs des distalen Endabschnitts zum Implantationsort unmittelbar an der Stelle der Gewebereizung abgegeben werden, wodurch eine hohe therapeutische Effizienz erreicht wird. Weiterhin kann im Vergleich zu einem nicht verbreiterten distalen Endabschnitt des Sondenkörpers bei gleicher Wirkstoffmenge ein größeres Wirkstoffreservoir nahe bzw. am Implantationsort zur Verfügung gestellt werden.

Hier und im Weiteren wird durch die Begriffe "axial" und "radial" eine Richtung entlang der Erstreckungsrichtung des schlauchartigen Elektrodenkörpers (Schlauchseele) bzw. senkrecht zur Erstreckungsrichtung des Elektrodenkörpers bezeichnet.

Bei einer vorteilhaften Ausführungsform der erfindungsgemäßen Elektrodensonde ist die am distalen Ende des Sondenkörpers angeordnete Elektrode mit dem distalen Endabschnitt des Sondenkörpers zumindest in axialer Richtung mechanisch gekoppelt, beispielsweise indem sie mit dem distalen Endabschnitt verbunden ist und/oder diesen hintergreift, und die elektrische Zuleitung zur Elektrode ist relativ zum Sondenkörper in axialer Richtung bewegbar. Weiterhin ist der distale Endabschnitt des Sondenkörpers so ausgebildet, dass wenigstens ein Teil des distalen Endabschnitts bei einer beispielsweise manuell ausgeführten Relativbewegung zwischen der elektrischen Zuleitung und dem Sondenkörper durch eine von der Elektrode auf den distalen Endabschnitt übertragene Kraft zu einer radial verbreiterten Wulst verformbar ist. Der distale Endabschnitt weist zu diesem Zweck eine entsprechende Nachgiebigkeit auf. Durch diese Maßnahme kann eine erfindungsgemäße Elektrodensonde in besonders einfacher Weise realisiert werden, wobei die Elektrodensonde mit einem nicht verbreiterten distalen Endabschnitt implantiert und nach Implantieren am Implantationsort der distale Endabschnitt durch eine Relativbewegung zwischen der elektrischen Zuleitung und dem Sondenkörper in einfacher Weise radial verbreitert werden kann.

Bei einer vorteilhaften Ausgestaltung obiger Ausführungsform der erfindungsgemäßen Elektrodensonde ist eine den Sondenkörper wenigstens in einem Teilabschnitt umgebende Hülle angeordnet, die mit dem distalen Endabschnitt in axialer Richtung beispielsweise durch Verkleben mechanisch so gekoppelt ist, dass dieser durch eine Relativbewegung zwischen der elektrischen Zuleitung und der Hülle zu der radial verbreiterten Wulst verformbar ist. Durch eine solche Hülle kann eine Relativbewegung zwischen dem Sondenkörper und der axialen Zuleitung zur Wulstbildung im distalen Endabschnitt des Sondenkörpers in besonders einfacher Weise bewirkt werden.

Bei einer weiteren vorteilhaften Ausführungsform der erfindungsgemäßen Elektrodensonde ist die Elektrode in Form einer Einschraubelektrode zum Einschrauben in ein Körpergewebe, beispielsweise Herzgewebe, ausgebildet, welche durch Drehen ihrer elektrischen Zuleitung zwischen einer Passivstellung, in der sie sich innerhalb des Sondenkörpers befindet, und einer Aktivstellung, in der sie sich wenigstens teilweise außerhalb des Sondenkörpers befindet, verlagerbar ist. Die Verankerung der Elektrodensonde mittels einer Einschraubelektrode ist dem Fachmann an sich bekannt, beispielsweise aus der eingangs genannten Druckschrift DE 20 2006 020 517 U1, so dass hier nicht näher darauf eingegangen werden muss. Wesentlich hierbei ist, dass der distale Endabschnitt des Sondenkörpers so ausgebildet ist, dass wenigstens ein Teil des distalen Endabschnitts beim Einschrauben der Einschraubelektrode in das Körpergewebe durch eine vom Körpergewebe auf den Verformungsabschnitt ausgeübten Kraft zu einer radial verbreiterten Wulst verformbar ist. Der distale Endabschnitt weist zu diesem Zweck eine entsprechende Nachgiebigkeit auf. Durch diese Maßnahme kann eine Wulstbildung im distalen Endabschnitt des Sondenkörpers in besonders einfacher Weise beim Einschrauben der Einschraubelektrode erreicht werden. Insbesondere kann die Elektrodensonde mit einem nicht verbreiterten distalen Endabschnitt am Implantationsort positioniert und der distale Endabschnitt des Sondenkörpers während der Verankerung mittels Einschrauben der Einschraubelektrode im Körpergewebe in einfacher Weise radial verbreitert werden.

Bei einer vorteilhaften Ausgestaltung obiger Ausführungsform der erfindungsgemäßen Elektrodensonde ist die Einschraubelektrode im Zusammenwirken mit einem im Bereich des distalen Endabschnitts am Sondenkörper angebrachten Gegenlager ("Steigungsgeber") durch Drehen der Zuleitung zwischen der Passivstellung und der Aktivstellung verlagerbar. Die Anwendung eines solchen Steigungsgebers ist dem Fachmann an sich bekannt, beispielsweise aus der vorgenannten Druckschrift, so dass hier nicht näher darauf eingegangen werden muss. Wesentlich hierbei ist, dass der distale Endabschnitt so ausgebildet ist, dass er beim Drehen der Zuleitung durch eine von der Einschraubelektrode auf das Gegenlager übertragene Kraft zu der radial verbreiterten Wulst verformbar ist. Durch diese Maßnahme kann eine Wulstbildung im distalen Endabschnitt des Sondenkörpers in besonders einfacher Weise selbsttätig beim Drehen der Zuleitung bewirkt werden, wobei nicht nur der vom Körpergewebe auf den distalen Endabschnitt bewirkte Druck sondern auch die vom Gegenlager auf den distalen Endabschnitt übertragene Kraft zur Wulstbildung beiträgt. Insbesondere kann die Elektrodensonde mit einem nicht verbreiterten distalen Endabschnitt am Implantationsort positioniert und der distale Endabschnitt des Sondenkörpers während der Verankerung mittels Einschrauben der Einschraubelektrode im Körpergewebe und Kraftübertragung auf das Gegenlager in einfacher Weise radial verbreitert werden.

Bei einer vorteilhaften Ausgestaltung obiger Ausführungsformen der erfindungsgemäßen Elektrodensonde ist der distale Endabschnitt des Sondenkörpers zumindest teilweise mit Lamellen formenden Durchbrechungen versehen, wobei die Lamellen sich in axialer Richtung erstrecken oder eine Erstreckungsrichtung mit zumindest einer in axialer Richtung gerichteten Komponente haben. Durch diese Maßnahme kann eine besonders gute Verformbarkeit des distalen Endabschnitts bei gleichzeitig relativ großer radialer Verbreiterung erreicht werden.

Bei einer weiteren vorteilhaften Ausführungsform der erfindungsgemäßen Elektrodensonde ist der distale Endabschnitt des Sondenkörpers mit einer Mehrzahl, beispielsweise in Umfangsrichtung auf dessen Außenfläche angeordnete, Verbreiterungsteile versehen, die zwischen einer Passivstellung, in der sie dem distalen Endabschnitt anliegen, und einer Aktivstellung, in der sie vom radialen Endabschnitt in radialer Richtung abstehen, bewegbar sind. Hierbei sind die Verbreiterungsteile so mit einem mechanischen Vorspannmittel, beispielsweise einem Federmittel, gekoppelt, dass sie in ihre Aktivstellung vorgespannt sind. Bei dieser Ausführungsform der erfindungsgemäßen Elektrodensonde kann diese beispielsweise mithilfe eines hülsenförmigen Einführwerkzeugs, durch das die Verbreiterungsteile in deren Passivstellung gezwungen sind, implantiert werden, wobei das Einführwerkzeug von der implantierten Elektrodensonde nach dem Implantieren entfernt wird, wodurch die Verbreiterungsteile durch das Vorspannmittel in deren Aktivstellung gebracht werden.

Bei einer vorteilhaften Ausgestaltung aller obiger Ausführungsformen der erfindungsgemäßen Elektrodensonde ist der distale Endabschnitt des Sondenkörpers auf seiner Außenfläche mit wenigstens einem Wirkstoff zur Abgabe in das Körpergewebe versehen, was beispielsweise durch eine Beschichtung der Außenfläche des distalen Endabschnitts erfolgen kann. Durch diese Maßnahme kann in besonders vorteilhafter Weise eine gezielte lokale Wirkstoffabgabe nahe bzw. in Kontakt mit dem Körpergewebe am Implantationsort erreicht werden, wodurch die therapeutische Wirksamkeit des abgegebenen Wirkstoffs verbessert ist.

Bei einer weiteren vorteilhaften Ausgestaltung aller obiger Ausführungsformen der erfindungsgemäßen Elektrodensonde ist der distale Endabschnitt des Sondenkörpers mit einer mit wenigstens einem Wirkstoffreservoir verbundenen Abgabevorrichtung zum Abgeben wenigstens eines Wirkstoffs in das Körpergewebe versehen. Auch durch diese Maßnahme kann zur Verbesserung der therapeutischen Wirksamkeit eine gezielte lokale Wirkstoffabgabe nahe bzw. in Kontakt mit dem Körpergewebe am Implantationsort erreicht werden.

Bei einer weiteren vorteilhaften Ausgestaltung aller obiger Ausführungsformen der erfindungsgemäßen Elektrodensonde ist die Elektrode mit einer mit wenigstens einem Wirkstoffreservoir verbundenen Abgabevorrichtung zum Abgeben wenigstens eines Wirkstoffs in das Körpergewebe versehen. Durch diese Maßnahme kann in besonders vorteilhafter Weise eine gezielte lokale Wirkstoffabgabe nahe bzw. in Kontakt mit dem Körpergewebe am Implantationsort erreicht werden, wodurch die therapeutische Wirksamkeit des abgegebenen Wirkstoffs verbessert ist. In diesem Fall kann es insbesondere von Vorteil sein, wenn das wenigstens eine Wirkstoffstoffreservoir durch ein bewegbares Kompressionsmittel komprimierbar ist, um einen Transport des Wirkstoffs vom Wirkstoffreservoir zur Abgabevorrichtung zu bewirken. Vorteilhaft kann es sein, wenn das Kompressionsmittel zur Kompression des Wirkstoffreservoirs durch Quellen eines mit dem Kompressionsmittel mechanisch gekoppelten, quellfähigen Materials bewegbar ist. Im letztgenannten Fall kann es weiterhin von Vorteil sein, wenn eine Wandung eines Kompartments zur Aufnahme des quellfähigen Materials mit wenigstens einer für wässriges Fluid zum Quellen des quellfähigen Materials durchlässigen Öffnung versehen ist, die so ausgebildet ist, dass ein Öffnungsquerschnitt entlang der Bewegungsrichtung des Kompressionsmittels in Abhängigkeit von der Stellung des Kompressionsmittels veränderbar ist.

Weiterhin kann es in obigen Ausgestaltungen der Erfindung, die mit einem Wirkstoffreservoir versehen sind, vorteilhaft sein, wenn das Wirkstoffreservoir im Sondenkörper aufgenommen ist.

Darüber hinaus kann es in obigen Ausgestaltungen der Erfindung, die mit einer Abgabevorrichtung zur Abgabe wenigstens eines Wirkstoffs versehen sind, vorteilhaft sein, wenn die Abgabevorrichtung zum Abgeben wenigstens eines Wirkstoffs mit einer mit dem Wirkstoffreservoir fluidisch verbundenen, elektrisch betriebenen Pumpe zum Zuführen von Wirkstoff zur Wirkstoffabgabevorrichtung fluidisch verbunden ist.

Die Erfindung wird nun anhand von Ausführungsbeispielen näher erläutert, wobei Bezug auf die beigefügten Zeichnungen genommen wird. Gleiche bzw. gleich wirkende Elemente sind mit denselben Bezugszahlen bezeichnet. Es zeigen:
- Fig. 1A-1B: schematische perspektivische Ansichten eines ersten Ausführungsbeispiels der erfindungsgemäßen Elektrodensonde;
- Fig. 2A-2C: schematische perspektivische Ansichten einer Variante des ersten Ausfüh- rungsbeispiels der erfindungsgemäßen Elektrodensonde;
- Fig. 3A-3D: schematische Ansichten eines zweiten Ausführungsbeispiels der erfin- dungsgemäßen Elektrodensonde;
- Fig. 4A-4C: schematische Ansichten eines dritten Ausführungsbeispiels der erfindungs- gemäßen Elektrodensonde;
- Fig. 5A-5D: schematische Ansichten einer Variante des zweiten bzw. dritten Ausfüh- rungsbeispiels der erfindungsgemäßen Elektrodensonde;
- Fig. 6A-6F: schematische Ansichten verschiedener Varianten der Elektrodenspitze zur Veranschaulichung einer weiteren Ausgestaltung der erfindungsgemäßen Elektrodensonde.

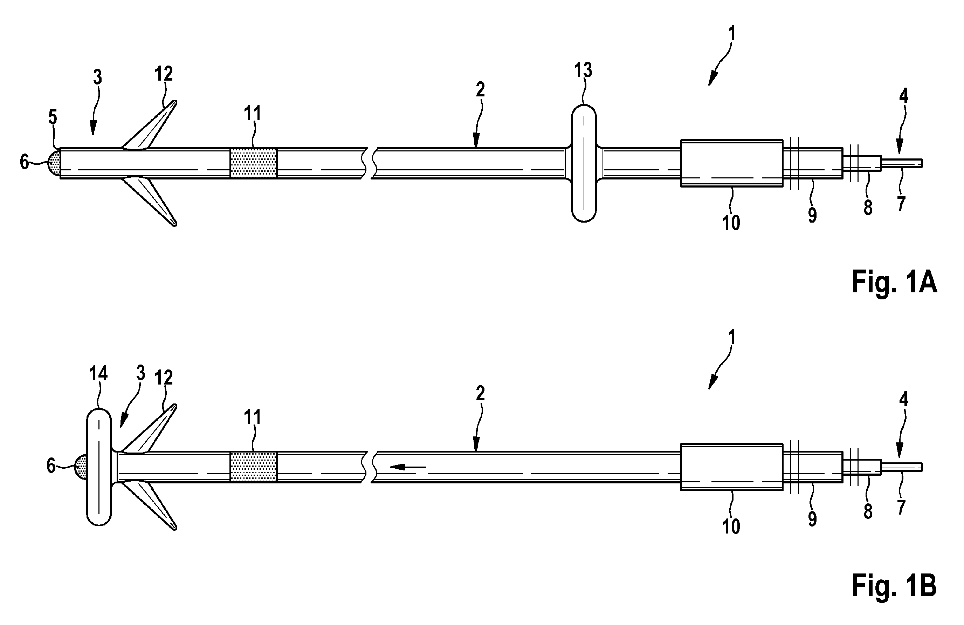

Seien zunächst die Figuren 1A und 1B betrachtet, worin in schematischen perspektivischen Ansichten ein erstes Ausführungsbeispiel der erfindungsgemäßen Elektrodensonde veranschaulicht ist.

Demnach umfasst eine hier beispielsweise als Herzschrittmacherelektrode ausgeführte, insgesamt mit der Bezugszahl 1 bezeichnete Elektrodensonde einen schlauchförmigen, biegsamen Sondenkörper 2 mit einem distalen Endabschnitt 3 und einem proximalen Endabschnitt 4, wobei der letztere einen (nicht dargestellten) in der Regel genormten Anschluss zum Verbinden der Elektrodensonde 1 mit einem (nicht dargestellten) Herzschrittmacher trägt. Eine axiale Richtung des Sondenkörpers 2 ist durch dessen Erstreckungsrichtung definiert, eine radiale Richtung des Sondenkörpers ist senkrecht hierzu orientiert.

An einer axialen Stirnfläche 5 bzw. am distalen Ende (des distalen Endabschnitts 3) des Sondenkörpers 2 ist eine zumindest annähernd halbkugelige Spitzenelektrode 6 angeordnet, welche das distale Ende des Sondenkörpers 2 radial hintergreift, so dass eine Kraft in axialer Richtung zwischen der Spitzenelektrode 6 und dem distalen Ende 3 des Sondenkörpers 2 übertragen werden kann. In einem vom Sondenkörper 2 geformten Lumen ist ein Innenleiter 7 und eine diesen umgebende erste Isolationshülle 8 aus einem isolierenden Material aufgenommen, welche gemeinsam als "Innenteil" des Sondenkörpers 2 aufgefasst werden können. Der Innenleiter 7, welcher beispielsweise in Form einer Drahtwendel ausgebildet ist, kontaktiert als elektrische Zuleitung die Spitzenelektrode 6.

Ein Außenleiter 9, welcher gleichermaßen beispielsweise in Form einer Drahtwendel ausgebildet ist und von einer nur teilweise dargestellten, zweiten Isolationshülle 10 umgeben ist, kontaktiert als elektrische Zuleitung eine proximal des distalen Endabschnitts 3 befindliche Ringelektrode 11. Wie in FIG. 1A beispielhaft dargestellt ist, kann der Außenleiter 9 in einem zwischen dem distalen Ende 3 und dem proximalen Ende 4 des Sondenkörpers 2 liegenden Bereich auf einen Block 13 gewendelt sein. Der Außenleiter 9 und die zweite Isolationshülle 10 können gemeinsam als "Außenteil" des Sondenkörpers 2 aufgefasst werden.

Wesentlich hierbei ist, dass der obig definierte Innenteil des Sondenkörpers 2 relativ zum obig definierten Außenteil des Sondenkörpers 2 in einem Bereich zwischen dem distalen Endabschnitt 3 und dem proximalen Endabschnitt 4 bewegbar ist. Andererseits ist sowohl im distalen Endabschnitt 3 des Sondenkörpers 2 die Spitzenelektrode 6 mit dem Sondenkörper 2 als auch im Bereich des proximalen Endabschnitts 4 der Innenteil des Sondenkörpers 2 mit dem Außenteil des Sondenkörpers 2 jeweils beispielsweise durch Verkleben verbunden.

Im Bereich des distalen Endabschnitts 3 sind auf der Außenfläche des Sondenkörpers 2 eine Mehrzahl im Umfangsrichtung verteilt angeordneter Verankerungselemente 12, die hier beispielsweise in Form von Spitzen ("Tines") ausgebildet und in einem Winkel von beispielsweise ca. 30° zum proximalen Endabschnitt 4 des Sondenkörpers 2 gerichtet sind, angeformt. Die Verankerungselemente 12 dienen in an sich bekannter Weise für eine so genannte passive Verankerung der Elektrodensonde 1 im ventrikulären Trabekelwerk.

Wie in FIG. 1B gezeigt und durch den Pfeil in Axialrichtung angedeutet ist, kann der distale Endabschnitt 3 des Sondenkörpers 2 durch Verschieben des Außenteils in distaler Richtung relativ zum Innenteil des Sondenkörpers 2 zu einer ringförmigen Wulst 14 verformt werden, die den Sondenkörper 2 und die Spitzenelektrode 6 radial verbreitert. Die Wulst 14 liegt unmittelbar benachbart zur Spitzenelektrode 6. Der distale Endabschnitt 3 des Sondenkörpers 2 ist zu diesem Zweck entsprechend nachgiebig ausgebildet.

Die Außenfläche des distalen Endabschnitts 3 des Sondenkörpers 2 ist mit wenigstens einem pharmazeutisch wirksamen Stoff (Wirkstoff) beschichtet, der an das umgebende Herzgewebe abgegeben werden kann. Der distale Endabschnitt 3 besteht zu diesem Zweck beispielsweise aus einem biokompatiblen Polymer, wie Silikonkautschuk und Polyurethan, oder aus einem hiervon verschiedenen resorbierbaren Material, das den Wirkstoff beim Abbau verzögert freisetzt. Im Prinzip kann der distale Endabschnitt 3 mit jedem gewünschten Wirkstoff wahlfrei beschichtet werden. Erfindungsgemäß bevorzugt sind entzündungshemmende Steroide (Glucocorticoide), insbesondere Alclomethason, Amcinonid, Beclomethason, Betamethason, Budenosid, Ciclesonid, Clobetasol, Clobetason, Clocortolon, Cloprednol, Cortisol, Cortison, Deflazacort, Desonid, Desoximethason, Dexamethason, Diflorason, Diflucortolon, Fludroxycortid, Flumetason, Flunisolid, Fluocinolon, Fluocinonid, Fluocortin, Fluocortolon, Fluorometholon, Flupredniden, Fluticason, Halcinonid, Halometason, Hydrocortison, Medryson, Methylprednisolon, Mometason, Prednicarbat, Prednisolon, Prednison, Prednyliden, Rimexolon, Tixocortol, Triamcinolon sowie Derivate hiervon. Die Derivatisierung kann insbesondere erfolgen als Aceponat, Acetat, Acetatpropionat, Acetonid, Benzoat, Buteprat, Butyrat, Butyrat-propionat, Diacetat, Dihydrogenphosphat, Dipropionat, Ethyl carbonat propionat, Hydrogensuccinat, Hexacetonid, Isonicotinat, Palmitat, Phosphat, Pivalat, Propionat, Natriumphosphat und Valerat. Der Wirkstoff kann insbesondere nur auf dem zum Herzgewebe gerichteten Bereich der Wulst 14 aufgebracht sein. Er kann insbesondere in einer Matrix gelöst oder suspendiert sein. Eine mögliche Dosierung liegt beispielsweise im Bereich von 0,01 bis 1000 mg.

Die in FIG. 1A und 1B schematisch dargestellte Elektrodensonde 1 kann mit einem "dünnen", d. h. nicht verbreiterten distalen Endabschnitt 3 des Sondenkörpers 2 in einfacher Weise implantiert werden. Nach Implantation kann durch beispielsweise manuelles Verschieben des Außenteils in distaler Richtung relativ zum Innenteil der distale Endabschnitt 3 zur ringförmigen Wulst 14 verformt werden, um durch die radiale Verbreiterung einen wirksamen Perforationsschutz für das Herzgewebe zu erreichen. Durch die ringförmige Wulst 14 kann wenigstens ein Wirkstoff, insbesondere ein Steroid, nahe bzw. in Kontakt mit dem Herzgewebe abgegeben werden, wodurch eine hohe therapeutische Wirksamkeit der eingesetzten Dosis erreicht werden kann.

Denkbar wäre auch, auf den Sondenkörper 2 eine Hülle aufzubringen, die mit dem distalen Endabschnitt 3 des Sondenkörpers 2 verbunden ist, um auf diese Weise den distalen Endabschnitt 3 zur Bildung der ringförmigen Wulst 14 relativ zum Innenteil in proximaler Richtung vorschieben zu können.

Eine Fixierung der Wulst 14 könnte beispielsweise durch eine (nicht dargestellte) Fixierhülse erfolgen, mit der die Elektrodensonde 1 nahe der Schrittmachertasche befestigt wird.

Denkbar wäre ebenso, im distalen Endabschnitt 3 die ringförmige Wulst 14 durch ein beispielsweise aus Nitinol bestehendes Federmittel zu erzeugen, wobei die Elektrodensonde 1 durch ein Einführbesteck implantiert wird, das nach dem Implantieren entfernt wird, so dass sich die Wulst unter Einwirkung des Federmittels ausbilden kann.

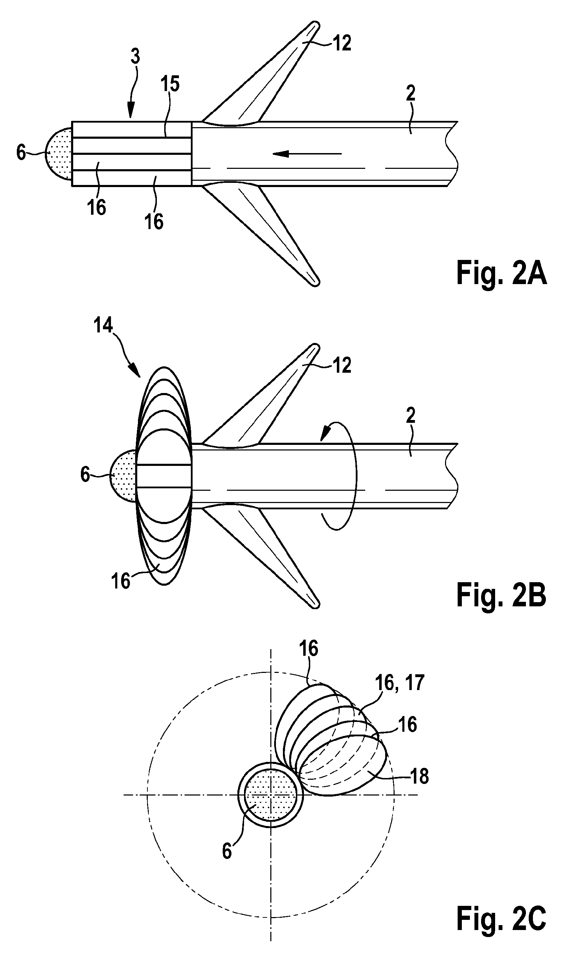

In den Figuren 2A bis 2C sind jeweils schematische perspektivische Ansichten zur Veranschaulichung einer vorteilhaften Variante des ersten Ausführungsbeispiels der erfindungsgemäßen Elektrodensonde 1 gezeigt. Um unnötige Wiederholungen zu vermeiden, werden lediglich die Unterschiede zu dem in den Figuren 1A und 1B gezeigten Ausführungsbeispiel erläutert und ansonsten wird Bezug auf die dort gemachten Ausführungen genommen. Die Figuren 2A und 2B zeigen eine perspektivische Seitenansicht des distalen Teils des Sondenkörpers 2, die Figur 2C eine Ansicht desselben von vom.

Demnach ist der distale Endabschnitt 3 des Sondenkörpers 2 mit einer Mehrzahl axialer Lamellen 16 versehen, die in Umfangsrichtung verteilt angeordnet sind. Zur Formung der Lamellen 16 sind zwischen diesen jeweils Durchbrechungen 15 ausgebildet. Wird das Außenteil relativ zum Innenteil in distaler Richtung verschoben, so formen die Lamellen 16 die Wulst 14. Aufgrund der lamellaren Struktur des distalen Endabschnitts 3 kann einerseits die Wulst 14 in einfacher Weise durch eine relative geringe Krafteinwirkung aufgestülpt werden, andererseits können die Lamellen 16 zu einer in radialer Richtung relativ breiten Wulst 14 verformt werden. Wie in Figur 2B durch den Pfeil angegeben, kann man durch Drehen des Sondenkörpers 2 um die Axialenrichtung zudem erreichen, dass nicht nur die Außenflächen 17 sondern auch die Innenflächen 18 der Lamellen 16 der Wulst 14 in Kontakt mit dem Herzgewebe gelangen. Werden die Innenflächen 18 der Lamellen 16 mit einem Wirkstoff beschichtet, so kann die zur Wirkstoffabgabe eingesetzte Oberfläche der Wulst 14, welche das Myokard kontaktiert, hierdurch weiter vergrößert werden, um auf diese Weise die therapeutische Effizienz des Wirkstoffs zu verbessern.

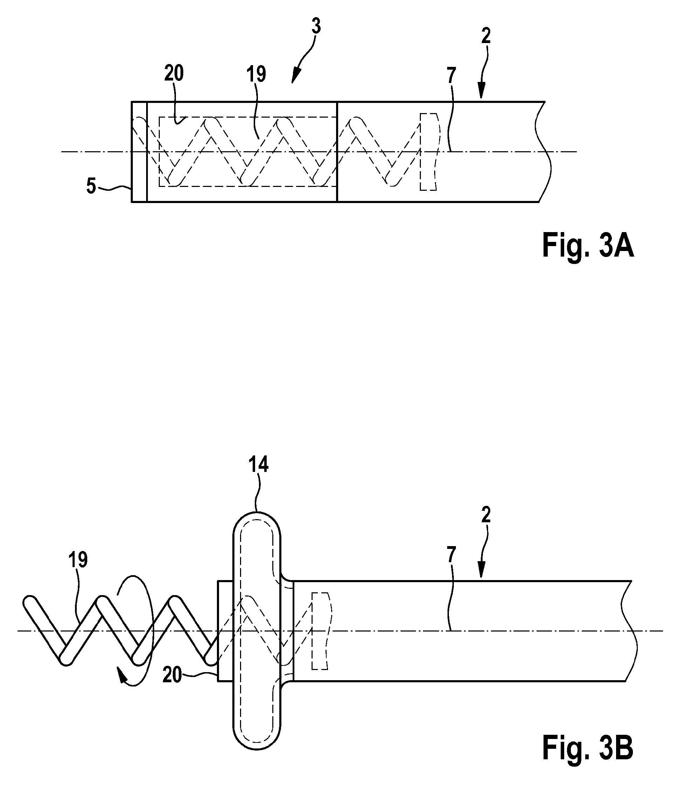

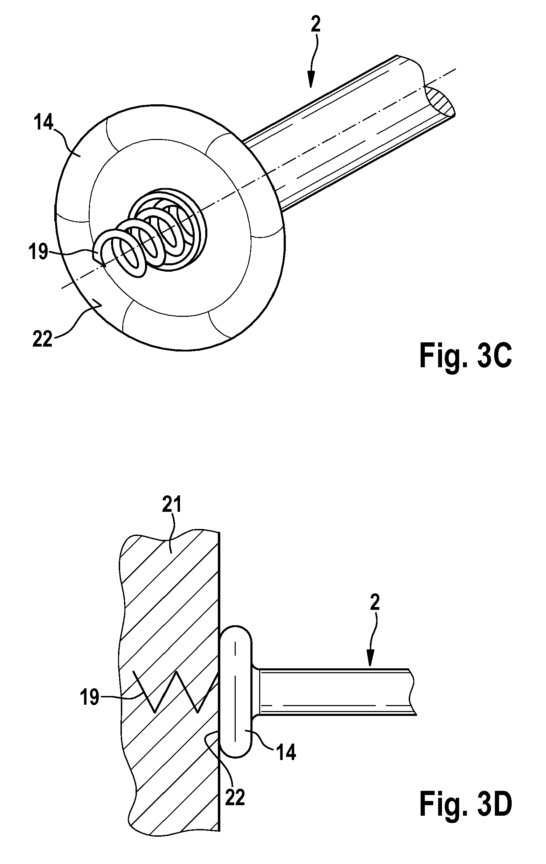

In den Figuren 3A bis 3D ist in schematischen Ansichten der distale Teil eines zweiten Ausführungsbeispiels der erfindungsgemäßen Elektrodensonde gezeigt. Um unnötige Wiederholungen zu vermeiden, werden lediglich die Unterschiede zu dem in den Figuren 1A und 1B gezeigten Ausführungsbeispiel erläutert und ansonsten wird Bezug auf die dort gemachten Ausführungen genommen. Die Figur 3A zeigt eine axiale Schnittansicht, die Figuren 3B-3D jeweils perspektivische Ansichten des distalen Teils des Sondenkörpers 2.

In der Elektrodensonde 1 gemäß dem zweiten Ausführungsbeispiel ist die am distalen Ende des Sondenkörpers 2 angeordnete Elektrode in Form einer korkenzieherartigen (d. h. helixförmigen) Einschraubelektrode 19 zum Einschrauben in das Myokard 21 ausgebildet. Die Einschraubelektrode 19 ist drehbar im Lumen des Sondenkörpers 2 gelagert und mit dem Innenleiter 7 drehfest verbunden. Der Innenleiter 7 weist genügend Torsionssteifigkeit auf, um ein Drehmoment auf die Einschraubelektrode 19 zum Einschrauben in das Myokard 21 zu übertragen. Die Einschraubelektrode 19 wirkt mit einem Gegenlager 20 (Steigungsgeber) zusammen, das auf einer Innenseite des distalen Endabschnitts 3 des Sondenkörpers 2 angebracht ist. Wird der Innenleiter 7 gedreht, so kann die Einschraubelektrode 19 zwischen einer in Figur 3A gezeigten Passivstellung, in der sie sich innerhalb des Sondenkörpers 2 befindet, und einer in Figur 3B gezeigten Aktivstellung, in der sie sich teilweise außerhalb des Sondenkörpers 2 befindet, verlagert werden. Die Einschraubelektrode 19 übt beim Herausdrehen aus dem Sondenkörper 2 eine in Axialrichtung zum proximalen Ende 4 des Sondenkörpers 2 gerichtete Kraft aus. Der Mechanismus zum Betätigen der Einschraubelektrode 19 ist dem Fachmann an sich bekannt, beispielsweise aus der oben genannten Druckschrift DE 20 2006 020 517 U1, so dass es sich erübrigt hier näher darauf einzugehen.

Wird nun die Elektrodensonde 1 am Implantationsort durch Einschrauben der Einschraubelektrode 19 im Herzgewebe 21 verankert, so übt das Gewebe eine Gegenkraft auf die distale Stirnfläche 5 des distalen Endabschnitts 3 des Sondenkörpers 2 aus. Gleichermaßen übt das Gegenlager 20 beim Herausdrehen der Einschraubelektrode 19 aus dem Sondenkörper 2 eine in Axialrichtung zum proximalen Ende 4 des Sondenkörpers 2 gerichtete Kraft auf den distalen Endabschnitt 3 aus. Beide Effekte tragen dazu bei, dass der distale Endabschnitt 3 beim Einschrauben der Einschraubelektrode 19 zu einer radial verbreiterten Wulst 14 verformt wird, wie in den Figuren 3B-3D schematisch veranschaulicht ist. Insbesondere ist in Figur 3D eine Situation dargestellt, bei der die Elektrodensonde 1 im Herzgewebe 21 verankert ist. Ersichtlich liegt die Wulst 14 bei verankerter Elektrodensonde 1 dem Herzgewebe 21 flächig an. Um eine Wulstbildung zu ermöglichen, weist der distale Endabschnitt 3 eine geeignete Verformbarkeit auf.

Die in den Figuren 3A bis 3D veranschaulichte Elektrodensonde 1 kann somit in komfortabler Weise mit einem schlauchförmigen distalen Endabschnitt 3 des Sondenkörpers 2 und der im Lumen des Sondenkörpers 2 aufgenommenen Einschraubelektrode 19 (siehe Figur 3A) implantiert werden. Durch Einschrauben der Einsschraubelektrode 19 am Implantationsort zum Verankern der Elektrodensonde 1 wird der distale Endabschnitt 3 des Sondenkörpers 2 zur ringförmigen, den Sondenkörper 2 radial verbreiternden Wulst 14 aufgestülpt, die dem Herzgewebe 21 flach anliegt (siehe Figur 3D). Die Wulst 14 wirkt somit als Perforationsschutz zum Verhindern einer Perforation des Herzgewebes 21 durch die Elektrodensonde 1.

Durch Beschichten einer dem Herzgewebe 21 zugewandten Außenfläche 22 der Wulst 14 mit einem Wirkstoff, beispielsweise Steroid, kann in direktem Kontakt mit der Gewebewand der Wirkstoff an das Herzgewebe 21 an der Stelle der Gewebereizung gezielt lokal abgegeben werden, so dass eine hohe therapeutische Effizienz erreichbar ist.

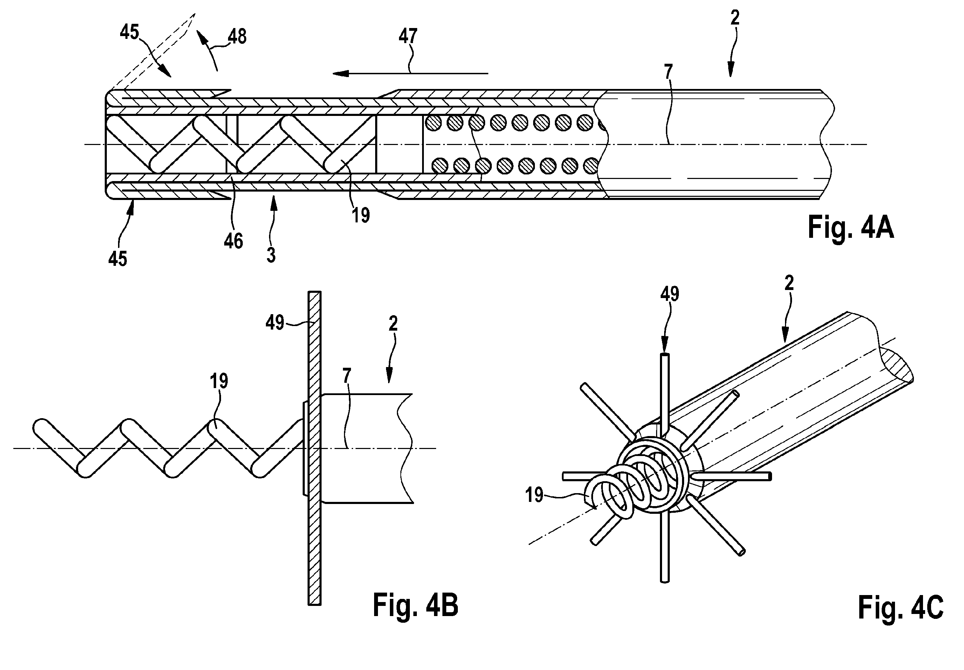

In den Figuren 4A bis 4C ist in schematischen Ansichten der distale Teil eines dritten Ausführungsbeispiels der erfindungsgemäßen Elektrodensonde gezeigt. Um unnötige Wiederholungen zu vermeiden, werden lediglich die Unterschiede zu dem in den Figuren 3A bis 3D gezeigten Ausführungsbeispiel erläutert und ansonsten wird Bezug auf die dort gemachten Ausführungen genommen. Die Figuren 4A und 4B zeigen jeweils eine axiale Schnittansicht, die Figur 4C eine perspektivische Ansichten des distalen Teils der Elektrodensonde 1.

In Figur 4A sind am schlauchförmigen distalen Endabschnitt 3 des Sondenköpers 2 in axialer Richtung anliegende Lamellen 45 radial verteilt angebracht, die an der äußersten distalen Spitze der Sonde scharnierartig befestigt sind.

Durch Einschrauben der Einsschraubelektrode 19 am Implantationsort zum Verankern der Elektrodensonde 1 wird eine Hülse 46 (Steigungsgeber) unter die Lammellen 45 geschoben (Pfeilrichtung 47), wodurch diese in radialer Richtung aufgerichtet werden (Pfeilrichtung 48). Die radial aufgerichteten Lamellen 49 liegen wie in den Figuren 4B und C gezeigt am Herzgewebe flach an. Die Hülse 46, die sich unter die Lamellen 45 schieben lässt, kann distal keilförmig angeformt werden, damit das Unterlaufen der Lamellen 45 besser unterstützt wird. Die Lamellen 45 können außen mit einem Wirkstoff beschichtet werden.

Alternativ kann durch verschieben des Außenteils gegen den Innenteil, wie gemäß den Figuren 1A und B erläutert, eine Hülse 46 (Steigungsgeber), die mit dem verschiebbaren Außenteil fest verbunden ist, unter die Lammellen 45 geschoben werden, wodurch diese in radialer Richtung aufgerichtet werden.

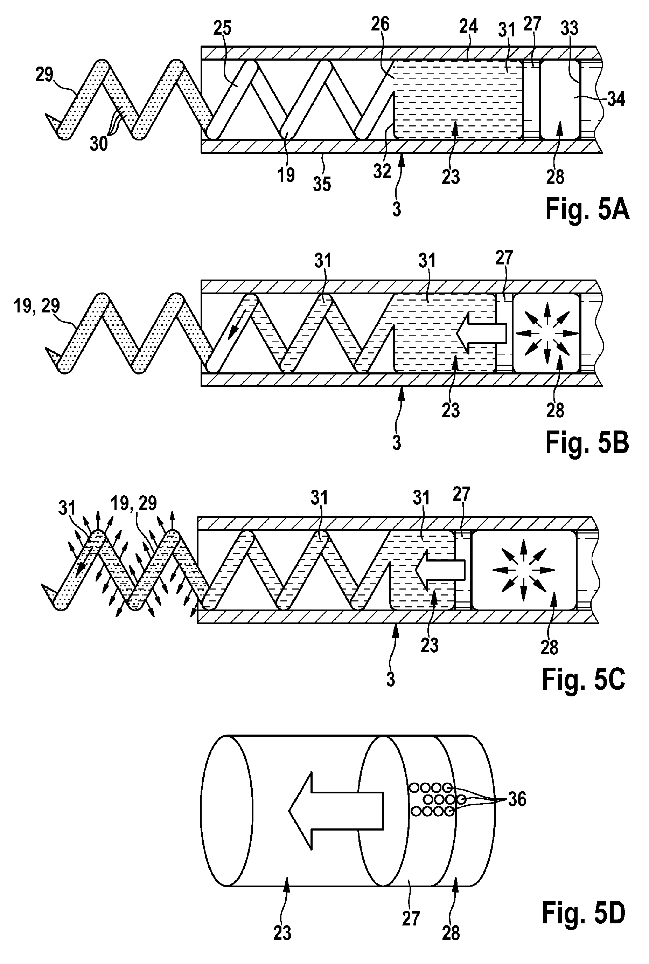

In den Figuren 5A bis 5D ist in schematischen Ansichten eine vorteilhafte Variante des zweiten bzw. dritten Ausführungsbeispiels der erfindungsgemäßen Elektrodensonde veranschaulicht. Demnach ist nicht nur die Wulst 14, sondern auch die Einschraubelektrode 19 zur Abgabe wenigstens eines Wirkstoffs geeignet ausgebildet. In den Figuren 5A bis 5D ist die Wulst 14 lediglich für eine einfachere Darstellung nicht dargestellt.

Zu diesem Zweck ist die Einschraubelektrode 19 mit einem Hohlraum 25 versehen, der mit einer Öffnung 26 einer ansonsten geschlossenen Gehäusewandung 24 fluidleitend verbunden ist. In einem distalen Elektrodenabschnitt 29 der Einschraubelektrode 19, welcher zum Verankern der Elektrodensonde 1 im Herzgewebe 21 dient, ist eine Vielzahl Poren 30 geformt, die in den Hohlraum 25 münden. Durch die Poren 30 kann ein Wirkstoff an die Umgebung, insbesondere im verankerten Zustand der Elektrodensonde 1 an das Herzgewebe 21, abgegeben werden.

Die Gehäusewandung 24 formt gemeinsam mit einem gegenüberliegend zu einer distalen Stirnfläche 32 der Gehäusewandung 24 angeordneten Druckkolben 27 ein Wirkstoffreservoir 23 zur Speicherung eines Wirkstoffs 31, beispielsweise Steroid, das über die Poren 30 der Einschraubelektrode 19 abgegeben werden kann. Der innerhalb der Gehäusewandung 24 in Axialrichtung bewegbar gelagerte Druckkolben 27, der das Wirkstoffreservoir 23 dichtend abschließt, wird auf seiner dem Wirkstoffreservoir 23 gegenüberliegenden Seite von einem quellfähigen Material 34 beaufschlagt. Als quellfähiges Material 34 kann beispielsweise Cellulose, Alginat, Stärke bzw. deren Derivate oder dergleichen eingesetzt werden. Das quellfähige Material 34 ist in einem vom Druckkolben 27 und der Gehäusewandung 24 geformten Kompartment 28 aufgenommen. Das Wirkstoffreservoir 23 und das Kompartment 28 für das quellfähige Material 34 sind im Lumen des Sondenkörpers 2 aufgenommen.

Wie in Figur 5B und Figur 5C veranschaulicht, kann das quellfähige Material 34 durch Aufnahme eines wässrigen Fluids, insbesondere Blut, unter Volumenzunahme quellen, wodurch der der Druckkolben 27 in distaler Richtung verlagert wird. Die hat zur Folge, dass das Wirkstoffreservoir 23 komprimiert und der Wirkstoff 31 zur Abgabe durch die Poren 30 in die Einschraubelektrode 19 gepresst wird. Die Gehäusewandung 24 bzw. der Außenmantel 35 des Sondenkörpers 2 sind zu diesem Zweck mit einer Vielzahl Öffnungen 36 versehen, durch die wässriges Fluid zum quellfähigen Material 34 gelangen kann. Die Öffnungen 36 sind hierbei so angeordnet, dass ein summarischer Öffnungsquerschnitt der Öffnungen 36 entlang der Bewegungsrichtung des Druckkolbens 27 in Abhängigkeit von der Stellung des Druckkolbens 27 gezielt veränderbar ist, um auf diese Weise den Fluideinstrom zu regeln.

Alternativ könnte der Druckkolben 27 auch über ein durch eine Druckmittelpumpe gefördertes Druckmittel hydraulisch beaufschlagt werden. Denkbar wäre auch, alternativ oder zusätzlich zur aktiven Druckbeaufschlagung des Wirkstoffreservoirs 23 eine passive, rein diffusive Wirkstoffabgabe vorzusehen. Ebenso könnte die Wirkstoffabgabe alternativ oder zusätzlich über ein elektrisches Potenzial, beispielsweise durch iontophoretischen Transport, erfolgen. Innerhalb des Wirkstoffreservoirs 23 kann der Wirkstoff 31 beispielsweise in reiner Form oder eingebettet in eine (gegebenenfalls quellbare) Matrix vorliegen.

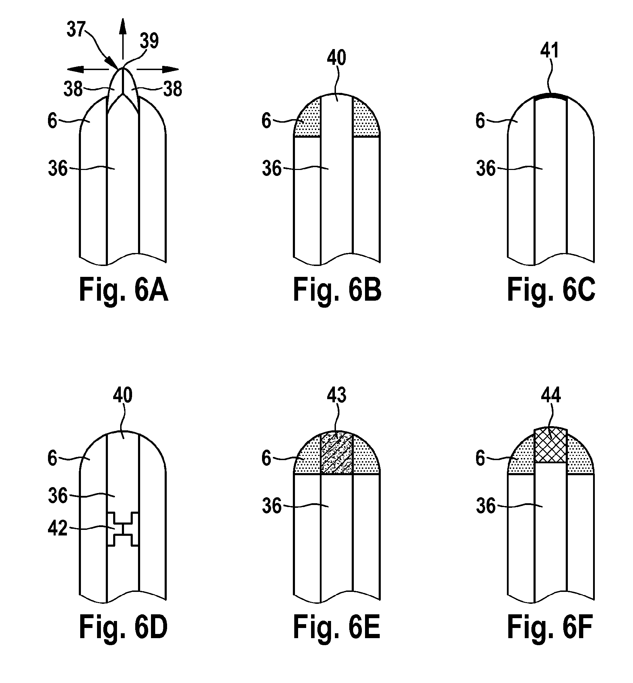

In den Figuren 6A bis 6F sind in schematischen Ansichten verschiedene Varianten des ersten Ausführungsbeispiels der erfindungsgemäßen Elektrodensonde veranschaulicht. Demnach ist nicht nur die Wulst 14, sondern auch die Spitzenelektrode 6 zur Abgabe wenigstens eines Wirkstoffs geeignet ausgebildet. In den Figuren 6A bis 6F ist die Wulst 14 lediglich für eine einfachere Darstellung nicht gezeigt. Zu diesem Zweck ist die Spitzenelektrode 6 mit einem Hohlraum 36 versehen, der als Wirkstoffreservoir für einen Wirkstoff dient und mit einer Fluidpumpe fluidleitend verbunden ist. Durch die Fluidpumpe kann der Wirkstoff von der Spitzenelektrode 6 abgegeben werden.

In der in Figur 6A gezeigten Variante ist am distalen Ende des Hohlraums 36 eine Dichtung 37 mit zwei Dichtlippen 38 angeordnet, die - wie durch die Pfeile angegeben ist - entgegen einer die beiden Dichtlippen 38 vorspannenden Kraft (beispielsweise Federkraft eines Federmittels) durch Druckbeaufschlagung des von der Fluidpumpe geförderten Fluids auseinander gedrückt werden können, um eine Passage 39 zur Abgabe des Wirkstoffs an die Umgebung freizugeben.

In der in Figur 6B gezeigten Variante ist der Hohlraum 36 mit einem offenen distalen Ende 40 versehen, so dass das Fluid bzw. Wirkstoff bei entsprechender Druckbeaufschlagung durch die Fluidpumpe an die Umgebung abgegeben werden kann.

In der in Figur 6C gezeigten Variante ist das distale Ende des Hohlraums 36 mit einer zur Wirkstoffabgabe geeigneten Membran 41 versehen, so dass Wirkstoff bei entsprechender Druckbeaufschlagung durch die Fluidpumpe an die Umgebung abgegeben werden kann. Die Membran 41 kann zu diesem Zweck beispielsweise als Porenmembran oder als geschlossene, eine Wirkstoffdiffusion ermöglichende Membran ausgeführt sein.

In der in Figur 6D gezeigten Variante ist in Entsprechung zur Figur 6A in einem Abstand vom distalen Ende des Hohlraums 36 eine Dichtung 42 angeordnet, die bei Druckbeaufschlagung durch das von der Fluidpumpe geförderte Fluid öffnen kann, um Wirkstoff durch das offene distale Ende 40 des Hohlraums 36 abzugeben.

In der in Figur 6E gezeigten Variante ist das distale Ende des Hohlraums 36 mit einem zur Wirkstoffabgabe geeigneten Stopfen 43 aus gesintertem Material versehen, so dass bei entsprechender Druckbeaufschlagung durch die Fluidpumpe ein Wirkstoff an die Umgebung abgegeben werden kann.

In der in Figur 6F gezeigten Variante ist das distale Ende des Hohlraums 36 mit einer Abdeckung 44 aus Drahtgaze versehen, so dass bei entsprechender Druckbeaufschlagung durch die Fluidpumpe ein Wirkstoff an die Umgebung abgegeben werden kann.

In den in den Figuren 6A bis 6F gezeigten Varianten wird eine Wirkstoffabgabe insbesondere durch eine im Herzschrittmacher aufgenommene Fluidpumpe gesteuert. Die Fluidpumpe kann hierbei über die Stromversorgung des Herzschrittmachers gespeist und über die elektronische Steuereinrichtung des Herzschrittmachers gesteuert werden. Über eine solche Anordnung kann die Abgabe von Wirkstoffen insbesondere gezielt an einen aktuellen Entzündungsstatus angepasst werden. Denkbar wäre auch, mehrere Wirkstoffe abzugeben, zu welchem Zweck beispielsweise eine Mehrzahl Hohlräume 36 in der Spitzenelektrode 6 vorzusehen wären. Gleichermaßen wäre es auch möglich, im Herzschrittmacher ein oder mehrere Wirkstoffreservoire vorzusehen, die fluidleitend mit dem wenigstens einen Hohlraum 36 verbunden sind. Obgleich die in den Figuren 6A bis 6F gezeigten Varianten im Zusammenhang mit der Spitzenelektrode 6 erläutert wurden, können diese gleichermaßen bei einer Einschraubelektrode 19 vorteilhaft eingesetzt werden.

Der im Rahmen der vorliegenden Erfindung eingesetzte Wirkstoff ist bevorzugt aus den folgenden Arzneistoffklassen ausgewählt: antimikrobielle, antimitotische, antimyotische, antineoplastische, antiphlogistische, antiproliferative, antithrombotische und vasodilatatorische Mittel.

Besonders bevorzugte Wirkstoffe sind Triclosan, Cephalosporin, Aminoglycosid, Nitrofurantoin, Penicilline wie Dicloxacillin, Oxacillin sowie Sulfonamide, Metronidazol, 5-Fluoruracil, Cisplatin, Vinblastin, Vincristin, Epothilone, Endostatin, Verapamil, Statine wie Mevastatin, Cerivastatin, Atorvastatin, Simvastatin, Fluvastatin, Pitavastatin, Pravastatin, Rosuvastatin sowie Lovastatin, Angiostatin, Angiopeptin, Taxane wie Paclitaxel, Immunsuppressiva oder -modulatoren wie z.B. Rapamycin oder dessen Derivate wie Biolimus, Everolimus, Deforloimus, Novolimus, Methotrexat, Colchicin, Flavopiridol, Suramin, Cyclosporin A, Clotrimazol, Flucytosin, Griseofulvin, Ketoconazol, Miconazol, Nystatin, Terbinafin, Steroide, Sulfasalazin, Heparin und seinen Derivaten, Urokinase, PPack, Argatroban, Aspirin, Abciximab, synthetisches Antithrombin, Bivalirudin, Enoxoparin, Hirudin, r-Hirudin, Protamin, Prourokinase, Streptokinase, Warfarin, Flavonoiden wie 7,3',4'-trimethoxyflavon, Sartane sowie Dipyramidol, Trapidil sowie Nitroprusside.

Die Wirkstoffe werden einzeln oder kombiniert in gleicher oder unterschiedlicher Konzentration eingesetzt.

Wie anhand der Ausführungsbeispiele ausführlich erläutert ist, ermöglicht die vorliegende Erfindung durch eine radial verbreiterte Struktur im distalen Endabschnitt des Sondenkörpers einen Perforationsschutz für die am distalen Ende der Elektrodensonde angeordnete Elektrode. Eine therapeutisch effiziente, lokale Wirkstoffabgabe am Ort der Gewebereizung kann durch die Abgabe von Wirkstoffen über radial verbreiterte Struktur und/oder über die am distalen Ende der Elektrodensonde angeordnete Elektrode erreicht werden. Insbesondere bei Verwendung einer Einschraubelektrode erfolgt eine Traumatisierung des Herzgewebes durch das Eindringen der Einschraubelektrode, so dass sich eine Gewebsentzündung nach der Implantation vor allem um die Einschraubelektrode herum ausbildet. Durch eine Abgabe von entzündungshemmenden Wirkstoffen über die Einschraubelektrode ist der Wirkstoff bereits am Zielort platziert. Neben einer schnelleren Anflutung können insbesondere auch wesentlich höhere Wirkstoffkonzentrationen im Zielgewebe erreicht werden, da die Diffusion vom Endokard ins Myokard zeitverzögert eintritt. Darüber hinaus wird ein Abtransport des freigesetzten Wirkstoffs durch die Blutzirkulation im Herzen unterbunden. Dieser Abtransport des freigesetzten Wirkstoffs ist in der Regel in den ersten Tagen nach der Implantation am stärksten ausgeprägt, da die Elektrodenspitze noch frei liegt bzw. noch nicht eingewachsen ist. Gleichzeitig ist die Entzündung jedoch auf dem Höhepunkt.

## Patentansprüche

1. Elektrodensonde (1) zur medizinischen Anwendung, welche einen schlauchartigen, flexiblen Sondenkörper (2), eine am distalen Ende des Sondenkörpers (2) angeordnete Elektrode (6, 19), sowie eine im Sondenkörper (2) geführte elektrische Zuleitung (7) zur Elektrode (6, 19) umfasst, **dadurch gekennzeichnet, dass** zumindest ein Teil eines distalen Endabschnitts (3) des Sondenkörpers (2) durch einen mit diesem gekoppelten Bewegungsmechanismus von einem ersten Zustand in einen in Bezug auf den ersten Zustand radial verbreiterten zweiten Zustand überführbar ist.

2. Elektrodensonde (1) nach Anspruch 1, **dadurch gekennzeichnet, dass** die Elektrode (6, 19) mit dem distalen Endabschnitt (3) des Sondenkörpers (2) in axialer Richtung mechanisch gekoppelt ist und die elektrische Zuleitung (7) relativ zum Sondenkörper (2) in axialer Richtung bewegbar ist, wobei der distale Endabschnitt (3) des Sondenkörpers (2) so ausgebildet ist, dass wenigstens ein Teil des distalen Endabschnitts (3) bei einer Relativbewegung zwischen der elektrischen Zuleitung (7) und dem Sondenkörper (2) durch die von der Elektrode (6, 19) auf den distalen Endabschnitt (3) übertragene Kraft zu einer radial verbreiterten Wulst (14) verformbar ist.

3. Elektrodensonde (1) nach einem der Ansprüche 1 bis 2, **gekennzeichnet durch**, eine den Sondenkörper (2) wenigstens in einem Abschnitt umgebende Hülle, welche mit dem distalen Endabschnitt (3) in axialer Richtung mechanisch so gekoppelt ist, dass dieser **durch** eine Relativbewegung zwischen der elektrischen Zuleitung (7) und der Hülle zu der radial verbreiterten Wulst (14) verformbar ist.

4. Elektrodensonde (1) nach Anspruch 1, bei welcher die Elektrode in Form einer Einschraubelektrode (19) zum Einschrauben in Körpergewebe (21) ausgebildet ist, die durch Drehen der Zuleitung (7) zwischen einer Passivstellung, in der sie sich innerhalb des Sondenkörpers (2) befindet, und einer Aktivstellung, in der sie sich wenigstens teilweise außerhalb des Sondenkörpers (2) befindet, verlagerbar ist, **dadurch gekennzeichnet, dass** der distale Endabschnitt (3) des Sondenkörpers (2) so ausgebildet ist, dass wenigstens ein Teil des distalen Endabschnitts (3) beim Einschrauben der Einschraubelektrode (19) in das Körpergewebe (21) durch eine vom Körpergewebe (21) auf den distalen Endabschnitt (3) ausgeübte Kraft zu einer radial verbreiterten Wulst (14) verformbar ist.

5. Elektrodensonde (1) nach Anspruch 4, **dadurch gekennzeichnet, dass** die Einschraubelektrode (19) im Zusammenwirken mit einem im Bereich des distalen Endabschnitts (3) am Sondenkörper (2) angebrachten Gegenlager (20) durch Drehen der Zuleitung (7) zwischen der Passivstellung und der Aktivstellung verlagerbar ist, wobei der distale Endabschnitt (3) so ausgebildet ist, dass er beim Drehen der Zuleitung (7) durch eine von der Einschraubelektrode (19) auf das Gegenlager (20) übertragene Kraft zu der radial verbreiterten Wulst (14) verformbar ist.

6. Elektrodensonde (1) nach einem der Ansprüche 1 bis 5, **dadurch gekennzeichnet, dass** der distale Endabschnitt (3) des Sondenkörpers (2) zumindest teilweise mit Lamellen (16) formenden Durchbrechungen (18) versehen ist, wobei die Lamellen (18) sich in axialer Richtung erstrecken oder eine Erstreckungsrichtung mit zumindest einer in axialer Richtung gerichteten Komponente haben.

7. Elektrodensonde (1) nach Anspruch 1, **dadurch gekennzeichnet, dass** der distale Endabschnitt (3) des Sondenkörpers (2) mit einer Mehrzahl Verbreiterungsteile versehen ist, die zwischen einer Passivstellung, in der sie dem distalen Endabschnitt anliegen, und einer Aktivstellung, in der sie vom radialen Endabschnitt in radaler Richtung abstehen, bewegbar sind, wobei die Verbreiterungsteile so mit einem mechanischen Vorspannmittel gekoppelt sind, dass sie in ihre Aktivstellung vorgespannt sind.

8. Elektrodensonde (1) nach einem der Ansprüche 1 bis 7, **dadurch gekennzeichnet, dass** der distale Endabschnitt (3) des Sondenkörpers (2) wenigstens auf einem Teil seiner Außenfläche mit wenigstens einem Wirkstoff zur Abgabe in das Körpergewebe (21) versehen ist.

9. Elektrodensonde (1) nach einem der Ansprüche 1 bis 8, **dadurch gekennzeichnet, dass** der distale Endabschnitt (3) des Sondenkörpers (2) mit einer mit wenigstens einem Wirkstoffreservoir verbundenen Abgabevorrichtung zum Abgeben wenigstens eines Wirkstoffs in das Körpergewebe (21) versehen ist.

10. Elektrodensonde (1) nach einem der Ansprüche 1 bis 9, **dadurch gekennzeichnet, dass** die Elektrode (6, 19) mit einer mit wenigstens einem Wirkstoffreservoir (23) verbundenen Abgabevorrichtung (30) zum Abgeben wenigstens eines Wirkstoffs (31) in das Körpergewebe (21) versehen ist.

11. Elektrodensonde (1) nach Anspruch 10, **dadurch gekennzeichnet, dass** das wenigstens eine Wirkstoffstoffreservoir (23) durch ein bewegbares Kompressionsmittel (27) komprimierbar ist.

12. Elektrodensonde (1) nach Anspruch 11, **dadurch gekennzeichnet, dass** das Kompressionsmittel (27) zur Kompression des Wirkstoffreservoirs durch Quellen eines mit dem Kompressionsmittel mechanisch gekoppelten, quellfähigen Materials (34) bewegbar ist.

13. Elektrodensonde (1) nach Anspruch 12, **dadurch gekennzeichnet, dass** eine Wandung (24) eines Kompartments (28) zur Aufnahme des quellfähigen Materials (34) mit wenigstens einer für Fluid zum Quellen des quellfähigen Materials durchlässigen Öffnung (36) versehen ist, wobei Öffnungsquerschnitt entlang der Bewegungsrichtung des Kompressionsmittels (27) in Abhängigkeit von einer Stellung des Kompressionsmittels einstellbar ist.

14. Elektrodensonde (1) nach einem der Ansprüche 9 bis 13, **dadurch gekennzeichnet, dass** das Wirkstoffreservoir (23) im Sondenkörper (2) aufgenommen ist.

15. Elektrodensonde (1) nach Anspruch 9 oder 10, **dadurch gekennzeichnet, dass** die Abgabevorrichtung (37, 40-44) zum Abgeben wenigstens eines Wirkstoffs in das Körpergewebe mit einer mit dem wenigstens einen Wirkstoffreservoir fluidisch verbundenen, elektrisch betriebenen Pumpe zum Zuführen von Wirkstoff zur Wirkstoffabgabevorrichtung fluidisch verbunden ist.
